(19) Europäisches Patentamt
European Patent Office
Office européen des brevets

(11) Publication number: **0 447 116 B1**

(12) # EUROPEAN PATENT SPECIFICATION

(45) Date of publication of patent specification: **14.12.94**

(51) Int. Cl.[5]: **C07C 275/28**, C07C 273/18, A61K 31/17

(21) Application number: **91301868.5**

(22) Date of filing: **06.03.91**

(54) **Urea derivatives, their production, and pharmaceutical compositions containing them.**

(30) Priority: **12.03.90 JP 60754/90**

(43) Date of publication of application:
**18.09.91 Bulletin 91/38**

(45) Publication of the grant of the patent:
**14.12.94 Bulletin 94/50**

(84) Designated Contracting States:
**AT BE CH DE DK ES FR GB GR IT LI LU NL SE**

(56) References cited:
**EP-A- 0 097 273**
**EP-A- 0 335 375**
**EP-A- 0 399 422**
**DE-A- 2 928 485**
**US-A- 4 473 579**

(73) Proprietor: **YAMANOUCHI PHARMACEUTICAL CO. LTD.**
**No. 3-11 Nihonbashi-Honcho, 2-chome**
**Chuo-ku**
**Tokyo (JP)**

(72) Inventor: **Ito, Noriki**
**Angel Heim 2-503, 614 Ohmagi**
**Urawa-shi, Saitama, 336 (JP)**
Inventor: **Matsuda, Koyo**
**2-35-2-403, Kasuga**
**Tsukuba-shi, Ibaraki, 305 (JP)**
Inventor: **Iwaoka, Kiyoshi**
**5-9-303, Ninomiya 2-chome**
**Tsukuba-shi, Ibaraki, 305 (JP)**
Inventor: **Iizumi, Yuichi**
**2-35-2-406, Kasuga**
**Tsukuba-shi, Ibaraki, 305 (JP)**

(74) Representative: **Geering, Keith Edwin et al**
**REDDIE & GROSE**
**16 Theobalds Road**
**London WC1X 8PL (GB)**

EP 0 447 116 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to urea derivatives of the following general formula (I) and salts thereof, which are of value as drugs for the treatment and prevention of various diseases, related particularly to atherosclerosis

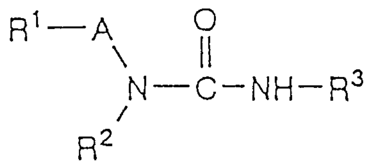

(I)

wherein $R^1$ is a condensed carbocyclic group containing at least 11 carbon atoms; $R^2$ is a cycloalkyl group-(which may have a bridgehead) ;$R^3$ is a tetrahydronaphthyl group or a phenyl group optionally substituted by one or more substituents selected from halogens and lower alkyl, amino, and mono- and di-lower alkylamino groups; and A is a single bond or an alkylene group (straight-chain or branched) containing 1 to 6 carbon atoms. The present invention further provides processes for producing these compounds, and pharmaceutical compositions containing them.

It is known that accumulation of cholesterol in the vascular system is an etiologic factor in various diseases such as coronary heart disease. Among these, atherosclerosis is a form of arteriosclerosis which is characterized by the deposition of lipids, (particularly cholesterol esters) on the walls, and the resulting thickening, of medium- and large-sized arteries.

It has recently been made clear that the production of such cholesterol ester is catalyzed by acyl-CoA cholesterol acyltransferase (ACAT). Thus, the excessive accumulation of cholesterol ester on the arterial wall is related to an increase in the ACAT enzyme level. Therefore, it is thought that if the ACAT enzyme is successfully inhibited, the esterification reaction of cholesterol will be retarded and the development and progression of atheromatous lesions due to excessive accumulation of cholesterol ester on the arterial wall will be prevented.

On the other hand, cholesterol in diets is absorbed as unesterified cholesterol, esterified by the action of ACAT in the body and released into the bloodstream in the form of chylomicrons. Therefore, inhibition of ACAT would suppress not only absorption of dietary cholesterol from the intestinal tract but also reabsorption of the cholesterol released into the intestine.

GB-A 2 113 684 discloses a series of antiatherosclerotic agents which are certain substituted urea and thiourea compounds having ACAT-inhibiting activity.

EP-A-0 335 375 also discloses a series of antihyperlipidemic and antiatherosclerotic agents which are certain substituted urea compounds having ACAT-inhibiting activity.

The compounds (I) according to the present invention are structurally different from the known compounds mentioned above and,as is demonstrated by the comparative pharmacological investigations described hereinafter, have pharmacological activity markedly superior to that of the known compounds.

EP-A-0 399 422, a document relevant under Article 54(3) and (4) EPC, discloses a series of urea derivatives having ACAT inhibiting activity.

The compounds (I) according to the present invention are structurally characterized in that a urea derivative is directly attached to a condensed carbocyclic nucleus with or without interposition of an alkylene group.

Referring to the definitions of the general formula (I), the "condensed carbocyclic group containing at least 11 carbon atoms" includes, among others,

fluorenyl 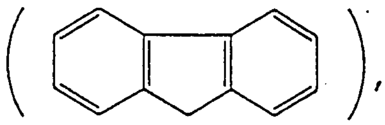,

anthracenyl 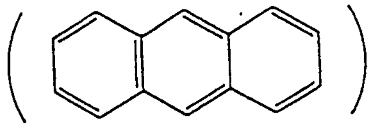 ,

dihydroanthracenyl 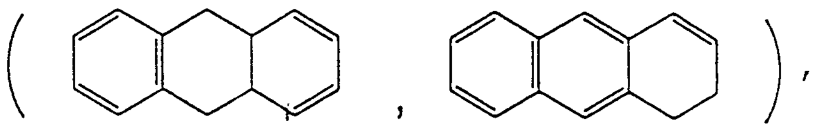,

phenanthrenyl 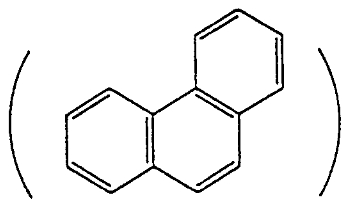 , 6,7,8,9-tetrahydro-5H-benzocycloheptenyl 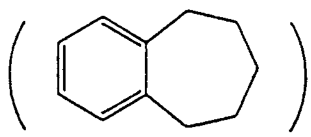 , 2,3-dihydro-1H-benz[+]-indenyl 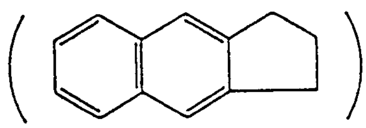 , 1H-benz[+]-indenyl 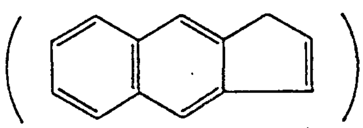, dibenzosuberanyl 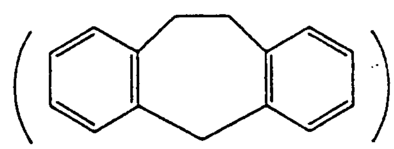 ,

as-indacenyl 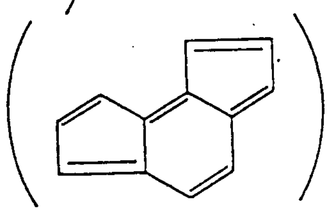 , s-indacenyl 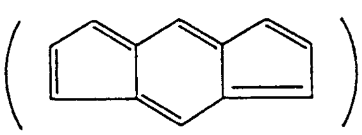 , acenaphthylenyl 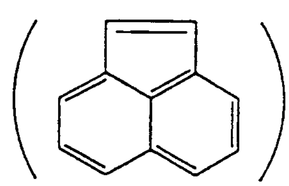 ,

phenarenyl 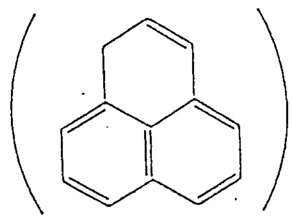, heptarenyl 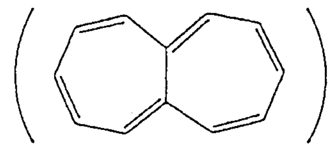,

biphenylenyl 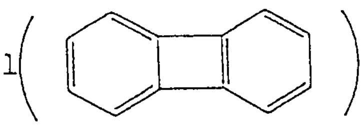

and so on.

The "cycloalkyl group", which may optionally have bridgeheads, may be a cycloalkyl group containing 3 to 18 carbon atoms, such as cyclopropyl, cyclobutyl, cyclopentyl, cyclohexyl, cycloheptyl, cyclooctyl, cyclononyl, cyclodecyl, cyclododecyl, cyclotridecyl, cyclopentadecyl, adamantyl, norbornyl and so on, and more preferably is a cycloalkyl group containing 6 to 10 carbon atoms.

Referring to the "phenyl group which may be substituted by one or more substituents selected from halogens and lower alkyl, amino, and mono- and di-lower alkylamino groups", the halogen may be chlorine, fluorine, bromine or iodine; the lower alkyl groups are straight-chain or branched alkyl groups containing 1 to 5 carbon atoms such as methyl, ethyl, propyl, isopropyl, butyl, isobutyl, sec-butyl, tert-butyl, pentyl (amyl), isopentyl, tert-pentyl, neopentyl, 1-methylbutyl, 2-methylbutyl, 1,2-dimethylpropyl, etc.; and the mono- and di-lower alkylamino groups are amino groups substituted by one or two lower alkyl groups as above.

One or more, whether the same or different, of these halogens and lower alkyl, amino, and mono- and di-lower alkylamino groups may be present on the phenyl group.

Such substituted phenyl groups include, among others, 2,4,6-trifluorophenyl, 2,6-dimethylphenyl, 2,6-diethylphenyl, 2,4,6-trimethylphenyl, 2,4,6-triethylphenyl, 4-propylphenyl, 2,6-diisopropylphenyl, 4-t-butylphenyl, 4-dimethylaminophenyl and so on.

The compounds (I) may form salts, which are included within the scope of the present invention. Among such salts are acid addition salts with inorganic acids, such as hydrochloric, hydrobromic hydroiodic, sulfuric, nitric and phosphoric acids,etc., and with organic acids, such as formic, acetic, oxalic, citric, succinic, fumaric, maleic, malic, tartaric, methanesulfonic and ethanesulfonic acids and so on.

The present invention also provides processes for producing the compounds (I) and salts thereof. Some representative processes are described below.

Process 1

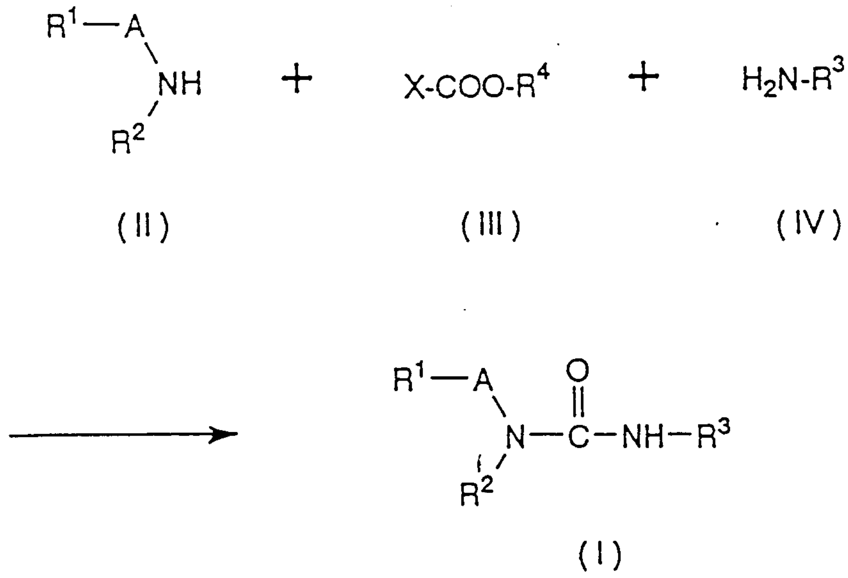

(II) (III) (IV)

(I)

In the above reaction formula, $R^1$, $R^2$, $R^3$ and A are as defined above, X represents a halogen atom and $R^4$ represents a phenyl group or a lower alkyl group.

The compound (I) can be prepared by reacting compounds (II), (III) and (IV) either concurrently or in any order - preferably by reacting amino compound (IV) with haloformic acid ester (III) and reacting the resulting carbamic ester with compound (II).

The haloformic acid esters (III) include isobutyl chloroformate, methyl chloroformate, methyl bromocarbonate, phenyl chloroformate and so on. There are cases in which the reaction can be advantageously hastened using a base such as potassium or sodium carbonate or hydroxide, triethylamine, N,N-dimethylaniline and so on.

The reaction solvent may be virtually any inert solvent, such as N,N-dimethylformamide, chloroform, benzene, toluene, xylene, dioxane, ether, tetrahydrofuran, chloroform, dichloromethane, dichloroethane and so on.

The reaction between amino compounds (IV) and (III) may be conducted under cooling or at room temperature and that between the resulting carbamic acid ester and compound (II) conducted at room temperature or under warming.

Process 2

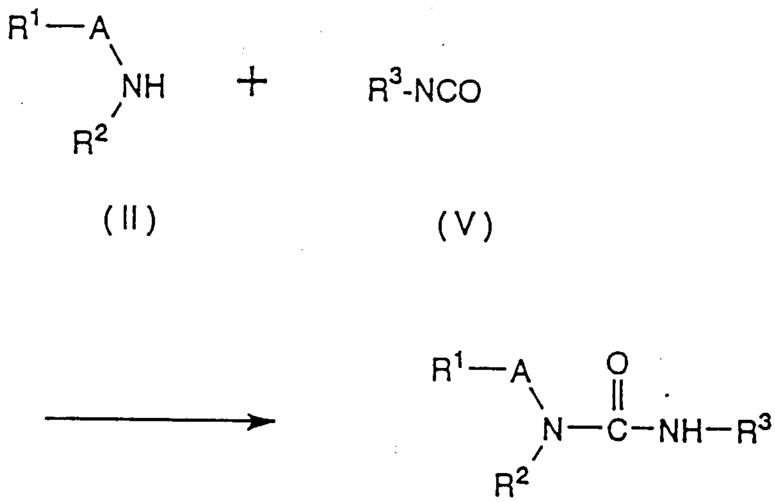

(II) (V)

(I)

In the above reaction formula, $R^1$, $R^2$, $R^3$ and A are as defined above; compound (I) is produced by reacting amino compound (II) and isocyanate compound (V), generally used in equimolar amounts.

This reaction can be conducted in an inert solvent, such as N,N-dimethylformamide, pyridine, benzene, toluene, dioxane, tetrahydrofuran, ether, chloroform, dichloromethane, dichloroethane, n-hexane, etc., at room temperature or with heating.

Process 3

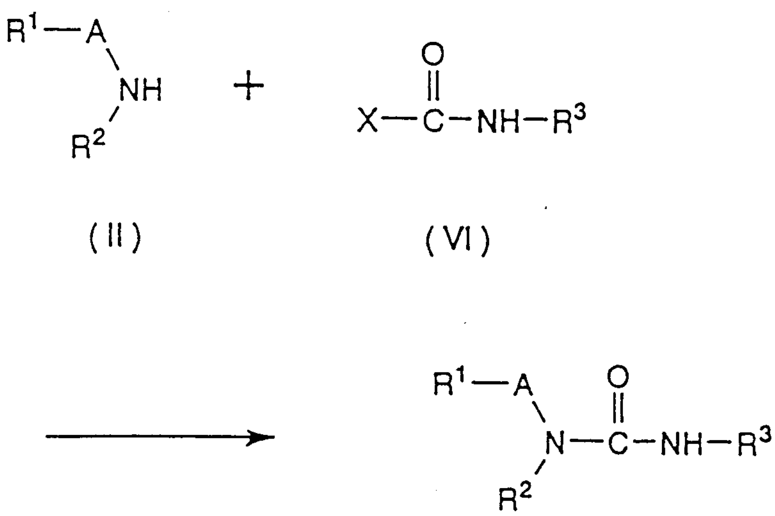

(II) (VI)

(I)

In the above reaction formula, $R^1$, $R^2$, $R^3$ and A are as defined above, and X represents a halogen atom. Compound (I) is produced by reacting amino compound (II) with halogen compound (VI).

This reaction can be conducted by reacting compounds (II) and (VI) in equimolar proportions in an inert solvent such as N,N-dimethylformamide, benzene, toluene, dioxane, tetrahydrofuran, ether, chloroform, dichloromethane, dichloroethane, n-hexane and so on. The reaction temperature is suitably decided depending on the starting compounds and the solvent used in the reaction, but the reaction is generally

carried out at room temperature or under warming.

The resulting compound (I) of the present invention can be isolated and purified in the free form or in the form of a salt thereof by salt-forming or desalting in conventional manner. Isolation and purification may involve extraction, crystallization, recrystallization, chromatography and/or other common chemical processes.

The compounds of the present invention inhibit ACAT to thereby inhibit the accumulation of cholesterol ester in the smooth muscle cells of the arterial wall. They also inhibit the absorption of cholesterol from the intestinal tract and facilitate the catabolism and excretion of cholesterol in the liver to thereby lower blood cholesterol levels and reduce the accumulation and storage of cholesterol ester in the arterial wall, which in turn inhibits the formation or progression of atherosclerotic lesions. These actions are not seen in the conventional lipid-lowering agents.

The compounds according to the present invention have been demonstrated by animal experiments to have excellent blood total cholesterol and low-density lipoprotein (LDL) lowering effects and are useful in lowering lipids as well as in the prevention and treatment of various diseases related to arteriosclerosis, such as cerebral infarction, transient ischemic attack, angina pectoris, peripheral thrombus and alteriosclerotic obliterans.

The effects of compounds of the present invention have been confirmed in the following manner.

i) ACAT enzyme inhibiting activity:

Inhibitory action against acyl CoA cholesterol acyltransferase (ACAT) activity in rabbit liver microsome

The rabbit liver microsome was prepared as an enzyme fraction according to the method of Heider (J. G. Heider et al., J. of Lipid Res., Vol. 24, 1127-34 (1983)).

To the mixture of 0.154 M phosphate buffer solution (pH 7.4), 2 mM dithiothreitol, 36 μM bovine serum albumin and 10-100 μg of microsome fraction was added liposome prepared by the method of Suckling (K. E. Suckling et al., FEBS Letters, Vol. 151, No. 1, 111-116 (1983)) so that the proportion of liposome became 20% v/v. To the mixture was added 2% v/v of each concentration of test compound solution in dimethyl sulfoxide and the mixture was heated at 37 °C for 5 minutes. Then 36 μM oleoyl CoA containing 1-$^{14}$C-oleoyl CoA was added and the resultant mixture was heated at 37 °C for 10 minutes. The reaction was stopped by adding chloroform/methanol (=2/1). After stirring, cholesterol oleate extracted into the chloroform layer was separated by thin layer chromatography and the radioactivity was determined as ACAT activity. The results obtained are shown in Table 1.

Table 1

| Test Compound | ACAT Inhibiting Activity $IC_{50}$* |
|---|---|
| Compound of Example 1 | $7.3 \times 10^{-8}$ M |
| Compound of Example 10 | $6.1 \times 10^{-8}$ M |

* $IC_{50}$: 50% Inhibition Concentration

ii) Lipid-lowering activity:

Male Sprague-Dawley rats, 5 weeks of age, were fed with a diet containing 1.5% cholesterol and 0.5% bile acid for 7 days and during the last 5 days, test compounds suspended in a 0.5% aqueous solution of methylcellulose were orally administered via sonde once a day. Two hours after the last administration, blood samples were collected under ether anesthesia for determination of serum total cholesterol level and HDL-cholesterol level. The cholesterol level was determined by the method of Siedel et al. (Siedel, J. et al., J. Clin. Chem. Clin. Biochem., 19, 838 (1981)) and the HDL-cholesterol level was determined by the method

of Ishikawa et al. (Ishikawa, T. et al., Lipids, 11, 628 (1976)). The results obtained are shown in Table 2.

Table 2

| Test Compound | Total Cholesterol $ED_{50}$ |
|---|---|
| Compound of Example 1 | 3.8 mg/kg |
| Compound of Example 10 | 1.7 mg/kg |
| Compound of Example 212 in GB-A-2,113,684 | 249 mg/kg |
| Compound of Example 1 in EP-A-0,335,375 | 514 mg/kg |

Pharmaceutical compositions can be manufactured by formulating compound (I) or salt thereof with a pharmaceutically acceptable carrier, vehicle or excipient commonly employed in the art in accordance with established pharmaceutical manufacturing practice.

Pharmaceutical compositions of the present invention can be administered orally in such dosage forms as tablets, pills, capsules, granules, powders, solutions, ect., parenterally in the form of an injectable preparation, or otherwise, for example in the form of suppositories. The dosage depends on the symptoms,the age and sex of the patient and other factors but generally the daily oral dose per adult human, for instance, is about 50 mg to about 500 mg, which can be administered in a single dose or in 2 to 4 divided doses.

The following examples are further illustrative of the present invention and should by no means be construed as defining the metes and bounds of the invention. In the examples, $^1$H-NMR stands for proton nuclear magnetic resonance spectrum, Mass for mass spectrum, and IR for infrared absorption spectrum.

Reference Examples are also given hereinafter for describing the processes for production of the starting compounds used in the Examples.

REFERENCE EXAMPLE 1

N-Cycloheptyl-[(2-fluorenyl)methyl]amine

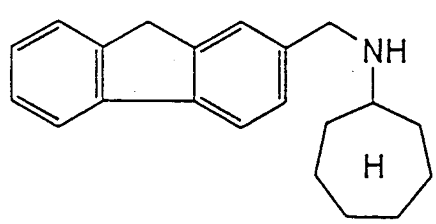

2-Formylfluorene (2.27 g, 11.7 mmol) and cycloheptylamine (1.39 g, 12.3 mmol) were heated together at 120 °C for 14 hrs. After cooling, the reaction mixture was distilled under reduced pressure. Then ethanol (30 ml) and sodium borohydride (0.44 g, 11.7 mmol) were added to the residue and the mixture was stirred for 0.5 hr. The mixture was then diluted with water (100 ml) and extracted with chloroform (80 ml x 2 times). The organic layer was dried over anhydrous magnesium sulfate. The solvent was then distilled off under reduced pressure to give 3.15 g of a pale yellow solid residue. $^1$H-NMR (δ ppm, in deuteriochloroform)

2.70 (1H, m), 3.79 (2H, s), 3.83 (2H, s)

Mass m/z 291 ($M^+$)

The following compounds were synthesized in generally the same manner as above.

REFERENCE EXAMPLE 2

N-Cycloheptyl-[(9-phenanthrenyl)methyl]amine

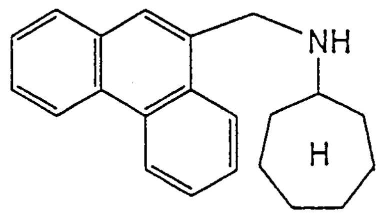

$^1$H-NMR (δ ppm, in deuteriochloroform)
2.84 (1H, m), 4.23 (2H, s), 8.65 (2H, m)

REFERENCE EXAMPLE 3

N-Cycloheptyl-(6,7,8,9-tetrahydro-5H-benzocyclohepten-5-yl)amine

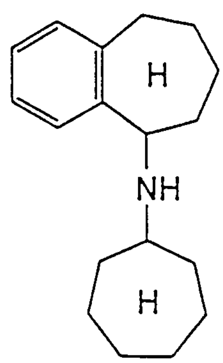

$^1$H-NMR (δ ppm, in deuteriochloroform)
3.95 (1H, m), 4.92 (1H, m), 7.10 (4H, m)

REFERENCE EXAMPLE 4

N-Cyclohepthyl-[(2-phenanthrenyl)methyl]amine

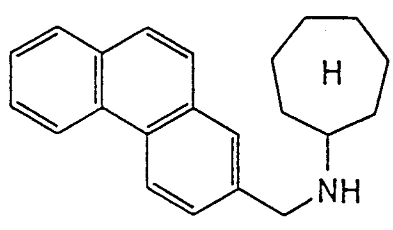

2-Methylphenanthrene (2.00 g, 10.4 mmol) was brominated with N-bromosuccinimide (2.05 g, 11.5 mmol) and the product compound was added gradually to a suspension of cycloheptylamine (2.38 g, 21.0 mmol) and potassium carbonate (2.90 g, 21.0 mmol) in dimethylformamide (20 ml) with ice-cooling. The mixture was then stirred at room temperature for 16 hrs and then filtered,and the filtrate was concentrated under reduced pressure. The residue was purified by silica gel column chromatography to give 2.11 g of a viscous liquid.

$^1$H-NMR (δ ppm, in deuteriochloroform)
2.83 (2H, m), 4.02 (2H, s), 8.68 (2H, m)

The following compound was synthesized in generally the same manner as above.

## REFERENCE EXAMPLE 5

N-Cycloheptyl-[(1-phenanthrenyl)methyl]amine

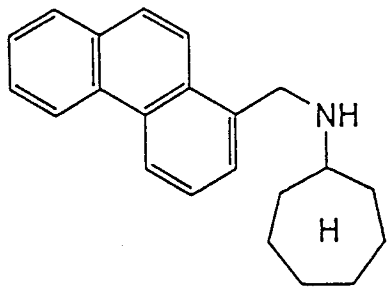

$^1$H-NMR (δ ppm, in deuteriochloroform)
2.83 (1H, m), 4.20 (2H, s), 8.62 (2H, m)
Mass m/z 303 ($M^+$)

## REFERENCE EXAMPLE 6

N-Cycloheptyl-2-fluoreneacetamide

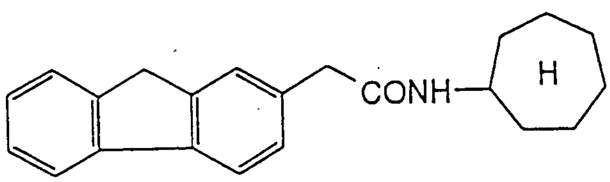

In dimethylformamide (50 ml) was dissolved 2-fluoreneacetic acid (2.24 g) followed by addition of 1-hydroxybenztriazole (2.0 g) and then dicyclohexylcarbodiimide (3.1 g) with constant stirring and ice-cooling. The mixture was further stirred at room temperature for 15 minutes. Cycloheptylamine (1.7 g) was then added under ice-cooling and the mixture was stirred at room temperature for 8 hrs. The resulting solid was filtered off and the filtrate was distilled under reduced pressure. The residue was extracted with 50 ml of chloroform and the extract was washed with 1N aqueous sodium hydroxide solution, 1N hydrochloric acid and water in the order mentioned and dried over anhydrous magnesium sulfate. The solvent was then distilled off under reduced pressure and the residue was purified by silica gel column chromatography to give 2.1 g of N-cycloheptyl-2-fluoreneacetamide as a solid.

$^1$H-NMR (δ ppm, in deuteriochloroform)
3.61 (2H, s), 3.90 (2H, s)

REFERENCE EXAMPLE 7

2-Cycloheptylaminoethylfluorene

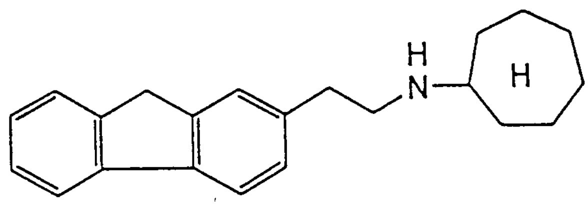

In dry tetrahydrofuran (30 ml) was dissolved 2-cycloheptylcarbamoylmethylfluorene (1.9 g) followed by dropwise addition of borane-methyl sulfide complex (1.8 ml) with ice-cooling. The mixture was then refluxed for 4 hrs, at the end of which time methanol (0.72 ml) was added with ice cooling. The mixture was stirred at room temperature for 30 minutes, followed by addition of concentrated hydrochloric acid (1.8 ml) with ice-cooling. The mixture was refluxed again for 30 minutes. The reaction mixture was then cooled with ice and the resulting solid was recovered by filtration and washed with ether. The solid matter thus obtained was dissolved in chloroform and the solution was alkalinized with aqueous sodium hydroxide solution. The chloroform layer was taken and dried and the solvent was distilled off under reduced pressure to give 2-cycloheptylaminoethylfluorene (1.2 g).

$^1$H-NMR (δ ppm, in deuteriochloroform)

2.88 (4H, s), 3.86 (2H, s)

REFERENCE EXAMPLE 8

N-Cycloheptyl-[(1-fluorenyl)methyl]amine

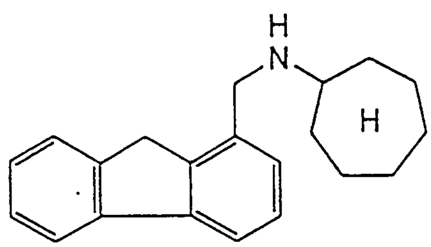

To N-cycloheptyl-1-fluorenecarboxamide (1.00 g, 3.28 mmol) was added a 1M solution of borane-tetrahydrofuran complex in THF (13 ml, 13 mmol) and the mixture was heated at 60 °C for 7.5 hrs. To this reaction mixture were added methanol (0.4 ml) and concentrated hydrochloric acid (3 ml) and the mixture was heated at 60 °C for 0.5 hr. Then, 1N aqueous sodium hydroxide solution (50 ml) was added at room temperature and the mixture was extracted with chloroform (80 ml x 2 times). The organic layer was taken, dried and concentrated. To the residue were added ether (30 ml) and 4N hydrogen chloride in ethyl acetate (2 ml) and the resulting white solid was collected by filtration. This solid was dissolved in chloroform (80 ml) and washed with 1N aqueous sodium hydroxide solution (80 ml x 1). The organic layer was dried and concentrated to give 0.90 g of a pale yellow solid.

$^1$H-NMR (δ ppm, in deuteriochloroform)

2.71 (1H, m), 3.84 (2H, s), 3.87 (2H, s)

Mass m/z 291 ($M^+$)

The following compound was synthesized in generally the same manner as above.

REFERENCE EXAMPLE 9

N-Cycloheptyl-[(4-fluorenyl)methyl)amine

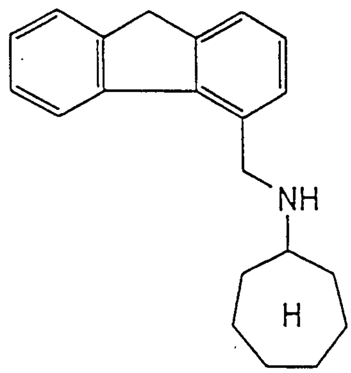

$^1$H-NMR (δ ppm, in deuteriochloroform)
2.86 (1H, m), 3.90 (2H, s), 4.20 (2H, s)

REFERENCE EXAMPLE 10

N-Cycloheptyl-[(2-biphenylenyl)methyl]amine

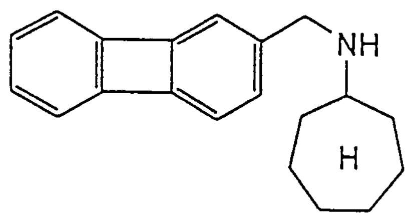

Biphenylenecarboxylic acid (1.00 g, 5.10 mmol) and a small amount of dimethylformamide were heated in 10 ml of thionyl chloride at 80 °C for 0.5 hr. After cooling, the solvent was distilled off. To the residue was added methylene chloride (30 ml), followed by gradual addition of a solution of cycloheptylamine (0.86 g, 7.6 mmol) and triethylamine (0.77 g, 7.6 mmol) in methylene chloride (20 ml) with ice-cooling. The mixture was stirred at room temperature for 1 hr, at the end of which time chloroform (50 ml) was added. The mixture was washed with water (50 ml), dried over anhydrous magnesium sulfate and concentrated under reduced pressure. The residue was purified by silica gel column chromatography to give 1.39 g of the amide as a white solid.
$^1$H-NMR (δ ppm, in deuteriochloroform)
1.58 (10H, s), 4.10 (1H, m)
Mass m/z 291 ($M^+$)

In a 1M solution of borane-tetrahydrofuran complex in THF (37 ml), 2.85 g of the above amide was heated at 65 °C for 8 hrs. To the solution was added methanol (1 ml) and concentrated hydrochloric acid (5 ml); the reaction mixture was further heated at 65 °C for 1 hr and diluted with 1N aqueous sodium hydroxide solution (100 ml). The mixture was extracted with chloroform (160 ml x 2 times) and the extract was dried over anhydrous magnesium sulfate and concentrated under reduced pressure. The residue was purified by silica gel column chromatography to give 1.19 g of the amine compound.
$^1$H-NMR (δ ppm, in deuteriochloroform)
2.88 (1H, m), 3.70 (2H, s)

REFERENCE EXAMPLE 11

N-(5,6,7,8-Tetrahydronaphthyl)-O-phenylcarbamate

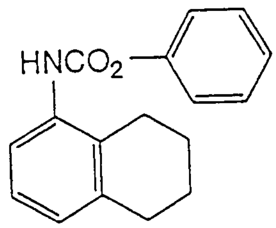

A solution of phenyl chloroformate (7.83 g, 50 mmol) in toluene (20 ml) was added gradually to a solution of 5,6,7,8-tetrahydronaphthylamine (7.36 g, 50 mmol) and triethylamine (6.07 g, 60 mmol) in toluene (100 ml) under ice-cooling. The mixture was then stirred at room temperature for 1 hr, followed by addition of ethyl acetate (100 ml). The mixture was washed with water (100 ml), dried over anhydrous magnesium sulfate and concentrated under reduced pressure. The residue was washed with hexane and the resulting white solid was collected by filtration to give 8.08 g of the carbamate.

$^1$H-NMR ($\delta$ ppm, in deuteriochloroform)

1.90 (4H, m), 2.70 (4H, m), 7.64 (1H, d)

Mass m/z 267 ($M^+$)

REFERENCE EXAMPLE 12

N-Cycloheptyl-1-(2-fluorenyl)ethylamine

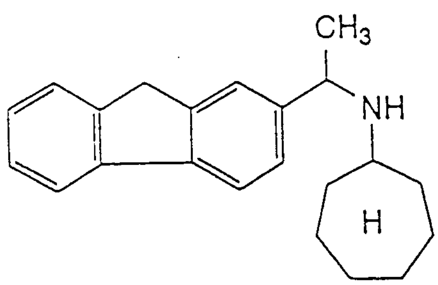

2-Acetylfluorene (4.17 g, 20 mmol) and cycloheptylamine (2.38 g, 21 mmol) were heated together at 130 °C for 14 hrs and then distilled under reduced pressure. To the residue were added ethanol (20 ml) and sodium borohydride (0.76 g, 20 mmol) and the mixture was stirred at room temperature overnight. The mixture was then diluted with water (100 ml) and extracted with chloroform (80 ml x 2 times). The organic layer was dried over anhydrous magnesium sulfate and concentrated. The residue was then purified by silica gel column chromatography to give 1.58 g of the amine compound.

$^1$H-NMR ($\delta$ ppm, in deuteriochloroform)

2.52 (1H, m), 3.76 (2H, s)

The following compound was synthesized in generally the same manner as above.

REFERENCE EXAMPLE 13

N-(Exo-2-norbornyl)-(9-phenanthrenyl)methylamine

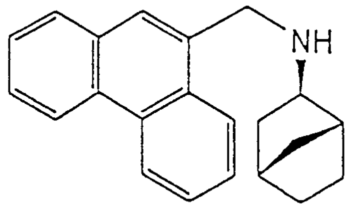

$^1$H-NMR (δ ppm, in deuteriochloroform)
2.28 (2H, m), 2.77 (1H, m), 4.20 (2H, m)
Mass m/z 301 ($M^+$)

EXAMPLE 1

1-Cycloheptyl-1-[(2-fluorenyl)methyl]-3-(2,4,6-trimethylphenyl)urea

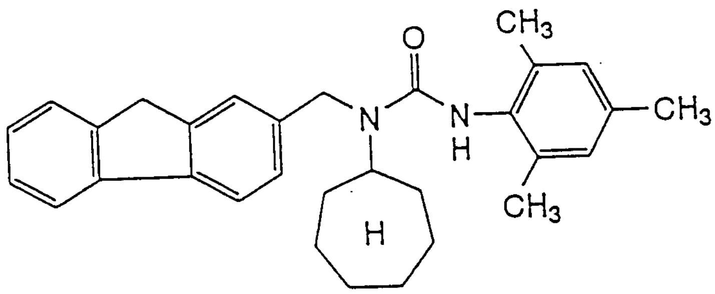

A mixture of N-cycloheptyl-[(2-fluorenyl)methyl]amine (980 mg, 3.36 mmol) and N-(2,4,6-trimethylphenyl)-o-phenylcarbamate (820 mg, 3.2 mmol) in toluene (10 ml) was refluxed for 15 hrs.

The reaction mixture was then diluted with toluene (50 ml) and washed with 1N aqueous sodium hydroxide solution (50 ml x 2 times). The organic layer was dried over anhydrous magnesium sulfate and concentrated. From the residue was obtained 940 mg of a white solid.

m.p. 124 - 126 °C
$^1$H-NMR (δ ppm, in deuteriochloroform)
1.96 (6H, s), 3.89 (2H, s), 4.58 (2H, s), 6.76 (2H, s)

| Elemental analysis (for $C_{31}H_{36}N_2O$) | | | |
|---|---|---|---|
| Found :<br>Calcd.: | C, 82.47%;<br>C, 82.26%; | H, 8.08%;<br>H, 8.02%; | N, 6.16%<br>N, 6.19% |

The following compounds were synthesized in generally the same manner as above.

EXAMPLE 2

1-Cycloheptyl-1-(6,7,8,9-tetrahydro-5H-benzocyclohepten-5-yl)-3-(2,4,6-trifluorophenyl)urea

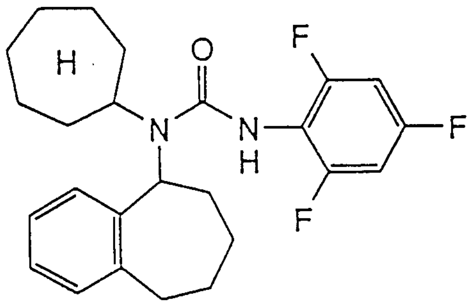

IR ($cm^{-1}$, KBr tablet) 1640, 1520, 1450, 1120
$^1H$-NMR (δ ppm, in deuteriochloroform)
4.43 (1H, m), 4.70 (1H, m), 6.67 (2H, m)
Mass (FAB) m/z 431 ($M^+$ + 1)

EXAMPLE 3

1-Cycloheptyl-1-[(9-anthracenyl)methyl]-3-(2,4,6-trifluorophenyl)urea

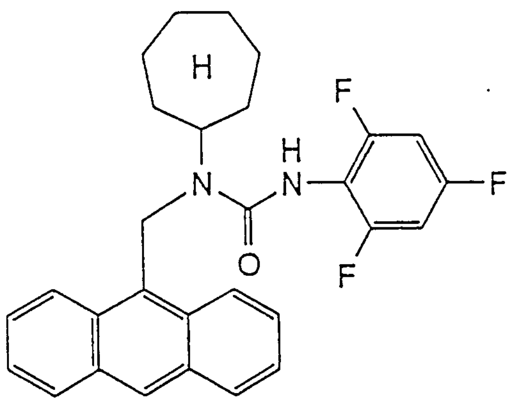

m.p. 175 - 177 °C
$^1H$-NMR (δ ppm, in deuteriochloroform)
3.21 (1H, m), 5.67 (2H, s), 8.48 (1H, s)

| Elemental analysis (for $C_{29}H_{27}N_2OF_3$) | | | | |
|---|---|---|---|---|
| Found : | C, 73.07%; | H, 5.81%; | N, 5.83%, | F, 11.97% |
| Calcd.: | C, 73.09%; | H, 5.71%; | N, 5.88%; | F, 11.96% |

EXAMPLE 4

1-Cycloheptyl-1-(9-fluorenyl)-3-(2,4,6-trifluorophenyl)urea

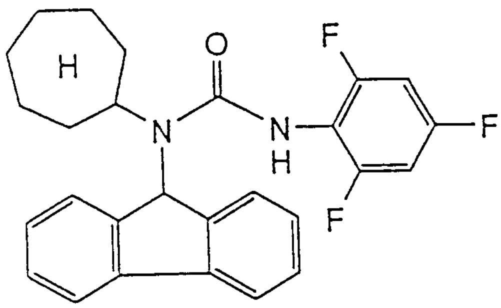

IR ($cm^{-1}$, KBr tablet) 1650, 1520, 1460, 1120
$^1$H-NMR (δ ppm, in deuteriochloroform)
4.62 (1H, m), 4.77 (1H, m), 6.42 (2H, m)
Mass (FAB) m/z ($M^+$ + 1)

EXAMPLE 5

1-Cycloheptyl-1-[(2-fluorenyl)methyl]-3-(2,4,6-trifluorophenyl)urea

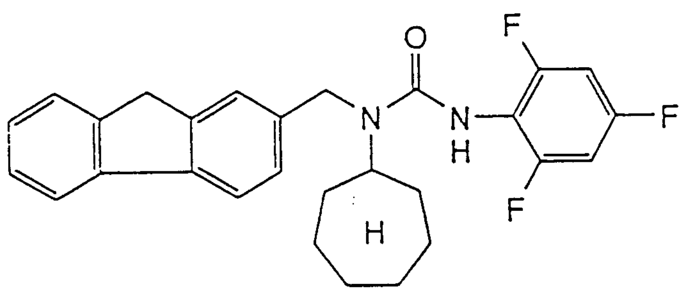

IR ($cm^{-1}$, KBr tablet) 1640, 1520, 1450, 1120
$^1$H-NMR (δ ppm, deuteriochloroform)
3.85 (2H, s), 4.35 (1H, m), 4.55 (2H,s) 6.54 (2H,m)
Mass (FAB) m/z 465 ($M^+$ + 1)

EXAMPLE 6

1-Cycloheptyl-1-[(9-phenanthrenyl)methyl]-3-(2,4,6-trifluorophenyl)urea

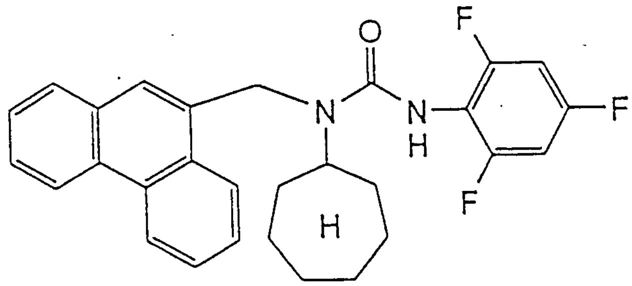

IR ($cm^{-1}$, KBr tablet) 1640, 1520, 1450, 1120
$^1$H-NMR (6 ppm, in deuteriochloroform)
4.50 (1H, m), 4.85 (2H, d), 6.51 (2H, m), 8.60 (2H, m)
Mass (FAB) m/z 477 ($M^+$ + 1)

EXAMPLE 7

1-Cycloheptyl-1-[(9-phenanthrenyl)methyl]-3-(4-propylphenyl)urea

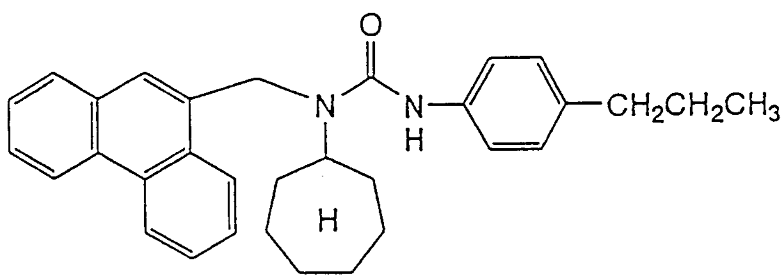

IR ($cm^{-1}$, KBr tablet) 1650, 1520, 1250, 750
$^1$H-NMR ($\delta$ ppm, in deuteriochloroform)
0.83 (3H, t), 2.42 (2H, t), 4.48 (1H, m), 4.88 (2H, s)
Mass (FAB) m/z 465 ($M^+$ + 1)

EXAMPLE 8

1-Cycloheptyl-1-[(2-fluorenyl)methyl]-3-(4-t-butylphenyl)urea

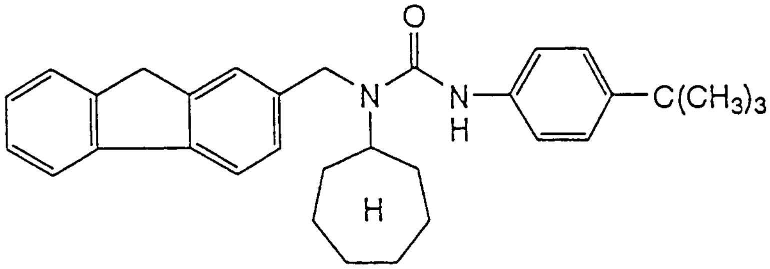

IR ($cm^{-1}$, KBr tablet) 1660, 1540, 1420, 1330
$^1$H-NMR (δ ppm, in deuteriochloroform)
1.23 (9H, s), 3.90 (2H, s), 4.54 (2H, s)
Mass (FAB) m/z 467 ($M^+$ + 1)

EXAMPLE 9

1-Cycloheptyl-1-[(2-fluorenyl)methyl]-3-(2,6-dimethylphenyl)urea

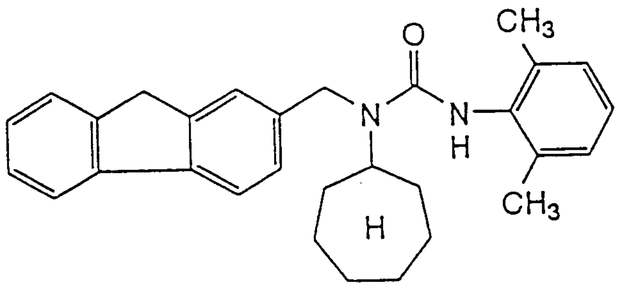

IR ($cm^{-1}$, KBr tablet) 1650, 1510, 1470, 760
$^1$H-NMR (δ ppm, in deuteriochloroform)
2.00 (6H, s), 3.90 (2H, s), 4.60 (2H, s), 6.95 (3H, s)

| Elemental analysis (for $C_{30}H_{34}N_2O$) | | | |
|---|---|---|---|
| Found : | C, 82.18%; | H, 7.94%; | N, 6.22% |
| Calcd.: | C, 82.15%, | H, 7.81%; | N, 6.39% |

EXAMPLE 10

1-Cycloheptyl-1-[(9-phenanthrenyl)methyl]-3-(2,4,6-trimethylphenyl)urea

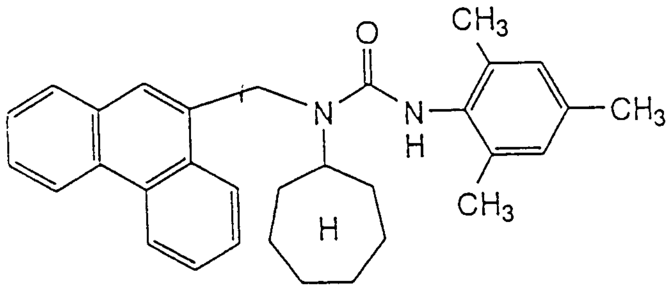

m.p. 108 - 110 °C
$^1$H-NMR (δ ppm, in deuteriochloroform)
2.06 (6H, s), 4.60 (1H, m), 5.00 (2H, s), 6.76 (2H, s)

| Elemental analysis (for $C_{32}H_{36}N_2O$) | | | |
|---|---|---|---|
| Found :<br>Calcd.: | C, 82.51%;<br>C, 82.72%; | H, 8.02%;<br>H, 7.81%; | N, 5.93%<br>N, 6.03% |

EXAMPLE 11

1-Cycloheptyl-1-[(2-fluorenyl)methyl]-3-(2,6-diethylphenyl)urea

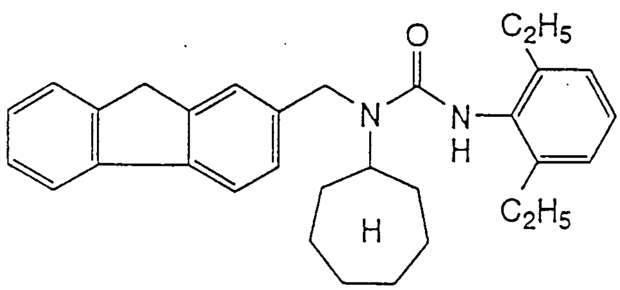

m.p. 134 - 135 °C
$^1$H-NMR (6 ppm, in deuteriochloroform)
1.09 (6H, t), 2.50 (4H, q), 4.00 (2H, s) 4.69 (2H, s)

| Elemental analysis (for $C_{32}H_{38}N_2O$) | | | |
|---|---|---|---|
| Found :<br>Calcd.: | C, 82.35%;<br>C, 82.36%; | H, 8.21%;<br>H, 8.21%; | N, 5.90%<br>N, 6.00% |

EXAMPLE 12

1-Cycloheptyl-1-[(2-fluorenyl)-methyl]-3-(2,6-diisopropylphenyl)urea

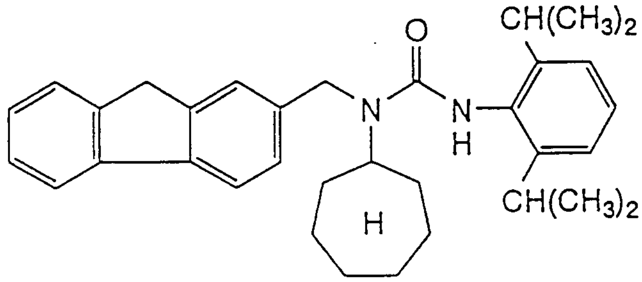

IR ($cm^{-1}$, KBr tablet) 1650, 1500, 1470, 1240
$^1$H-NMR (δ ppm, in deuteriochloroform)
2.98 (2H, m), 4.00 (2H, s), 4.70 (2H, s)
Mass (FAB) 495 (M + 1)

EXAMPLE 13

1-Cycloheptyl-1-[(2-phenanthrenyl)methyl]-3-(2,4,6-trifluorophenyl)urea

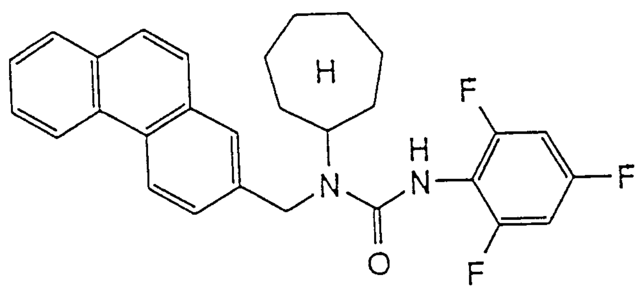

IR ($cm^{-1}$, KBr tablet) 1640, 1520, 1120, 750
$^1$H-NMR (δ ppm, in deuteriochloroform)
4.43 (1H, m), 4.74 (2H, s) 6.58 (2H, m)
Mass (FAB) m/z 477 ($M^+$ + 1)

EXAMPLE 14

1-Cycloheptyl-1-[(2-phenanthrenyl)methyl]-3-(2,4,6-trimethylphenyl)urea

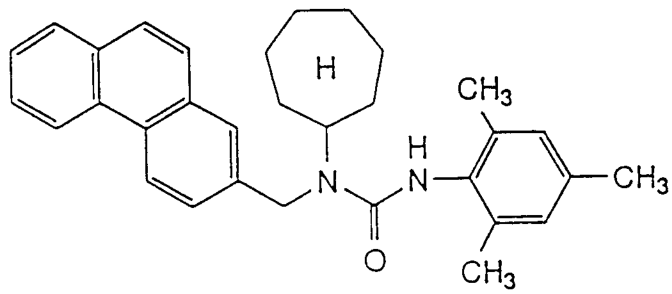

m.p. 120 - 122 °C
IR ($cm^{-1}$, KBr tablet) 1630, 1510, 1260, 810
$^{1}H$-NMR (δ ppm, in deuteriochloroform)
1.94 (6H, s), 4.70 (2H, s), 6.72 (2H, s)
Mass (FAB) m/z 465 ($M^{+}$ + 1)

EXAMPLE 15

1-Cycloheptyl-1-[(1-phenanthrenyl)methyl]-3-(2,4,6-trimethylphenyl)urea

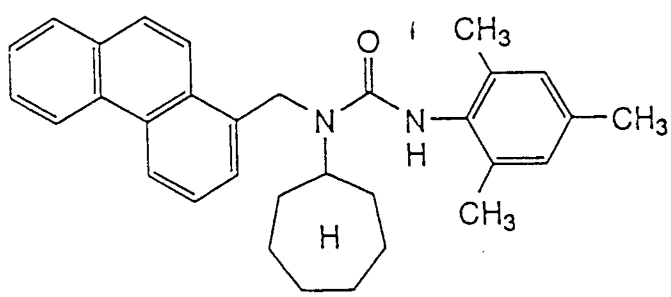

m.p. 163 - 165 °C
$^{1}H$-NMR (δ ppm, in deuteriochloroform)
2.06 (6H, s), 5.05 (2H, s), 6.77 (2H, s)

| Elemental analysis (for $C_{32}H_{36}N_{2}O$) | | | |
|---|---|---|---|
| Found : | C, 82.54%; | H, 7.97%; | N, 5.86% |
| Calcd.: | C, 82.72%; | H, 7.81%; | N, 6.03% |

EXAMPLE 16

1-Cycloheptyl-1-[(1-phenanthrenyl)methyl]-3-(2,4,6-trifluorophenyl)urea

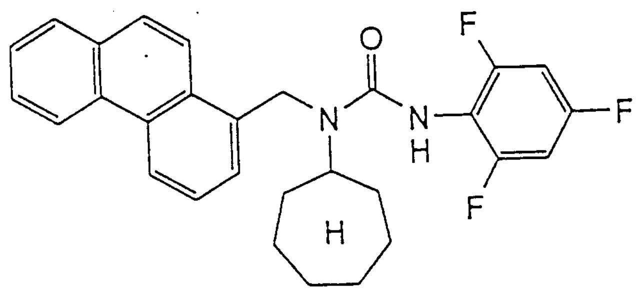

IR ($cm^{-1}$, KBr tablet) 1650, 1520, 1120, 750
$^1$H-NMR (δ ppm, in deuteriochloroform)
4.44 (1H, m), 5.05 (2H, s), 6.62 (2H, m)
Mass (FAB) m/z 477( $M^+$ + 1)

EXAMPLE 17

1-Cycloheptyl-[(1-fluorenyl)methyl]-3-(2,4,6-trimethylphenyl)urea

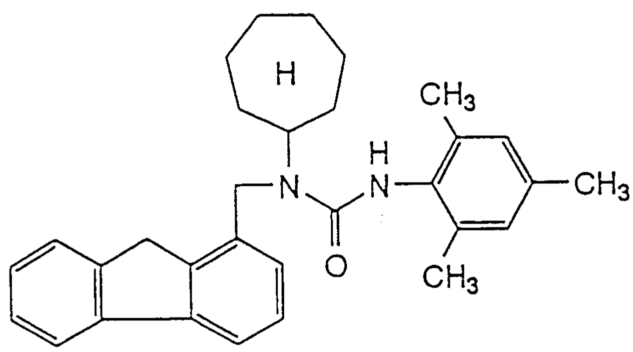

m.p. 183 - 184 °C
$^1$H-NMR (δ ppm, in deuteriochloroform)
2.00 (6H,s), 3.83 (2H, s), 4.58 (2H, s)

| Elemental analysis (for $C_{31}H_{36}N_2O$) | | | |
|---|---|---|---|
| Found : | C, 82.01%; | H, 8.06%; | N, 6.25% |
| Calcd.: | C, 82.26%; | H, 8.02%; | N, 6.19% |

EXAMPLE 18

1-Cycloheptyl-[(4-fluorenyl)methyl]-3-(2,4,6-trimethylphenyl)urea

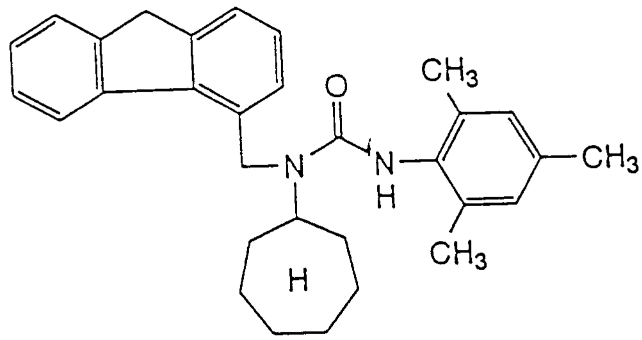

m.p. 133 - 136 °C
$^1$H-NMR (δ ppm, in deuteriochloroform)
2.04 (6H, s), 3.95 (2H, s), 4.97 (2H,s)

| Elemental analysis (for $C_{31}H_{36}N_2O$) | | | |
|---|---|---|---|
| Found : | C, 82.02%; | H, 8.14%; | N, 5.94% |
| Calcd.: | C, 82.26%; | H, 8.02%; | N, 6.19% |

EXAMPLE 19

1-Cycloheptyl-1-[(2-fluorenyl)ethyl]-3-(2,4,6-trimethylphenyl)urea

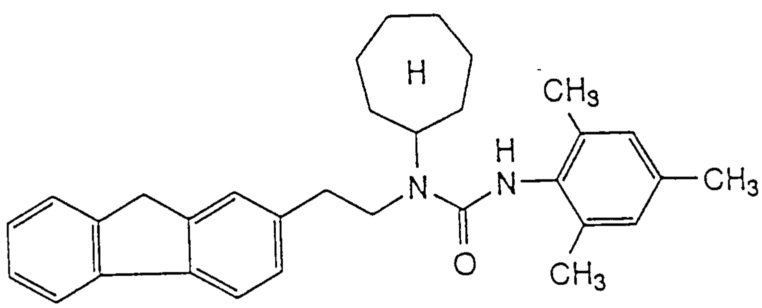

IR (cm$^{-1}$, KBr tablet) 2936, 1632, 1494
$^1$H-NMR (δ ppm, in deuteriochloroform)
2.12 (6H, s), 2.22 (3H, s), 3.00 (2H, t), 3.52 (2H, t), 3.84 (2H, s)
Mass (FAB) m/z 467 ($M^+$ + 1)

EXAMPLE 20

1-Cycloheptyl-1-[(2-fluorenyl)ethyl]-3-(2,4,6-trifluorophenyl)urea

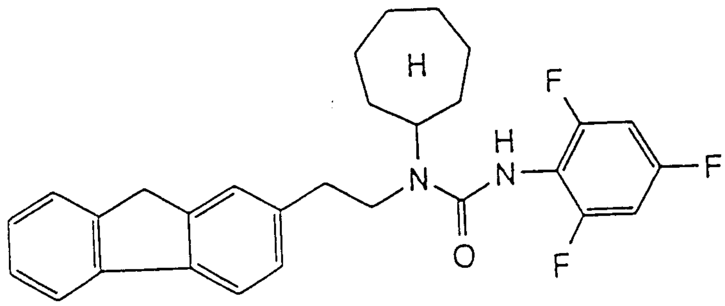

IR ($cm^{-1}$, Kbr tablet) 1638, 1522, 1452, 1120, 1044
$^{1}$H-NMR (δ ppm, in deuteriochloroform)
3.00 (2H, t), 3.52 (2H, t), 3.84 (2H, s)
Mass (FAB) m/z 479 ($M^{+}$ + 1)

EXAMPLE 21

1-Cycloheptyl-1-(6,7,8,9-tetrahydro-5H-benzocyclohepten-5-yl)-3-[p-(N,N-dimethylamino)phenyl]urea monohydrochloride

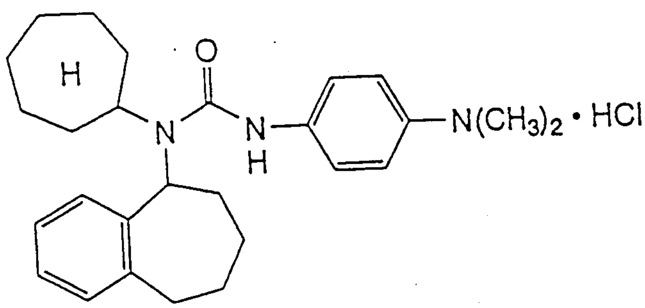

N-Cycloheptyl-(6,7,8,9-tetrahydro-5H-benzocycloheptenyl)amine (0.8 g, 3.1 mmol) and 4-(N,N-dimethylamino)phenylisocyanate (0.50 g, 3.1 mmol) was stirred in dichloromethane (10 ml) at room temperature for 18 hrs. The reaction mixture was then purified by silica gel column chromatography. The resulting urea compound was treated with hydrochloric acid in ether to give 0.72 g of the above hydrochloric compound.
IR ($cm^{-1}$, KBr tablet) 1660, 1520, 1320
$^{1}$H-NMR (δ ppm, in deuteriochoroform)
3.13 (6H, s), 4.40 (1H, m), 7.2-7.5 (8H,m)
Mass (FAB) m/z 421 ($M^{+}$ + 1)

EXAMPLE 22

1-Cycloheptyl-1-[(2-biphenylenyl)methyl]-3-(2,4,6-trimethylphenyl)urea

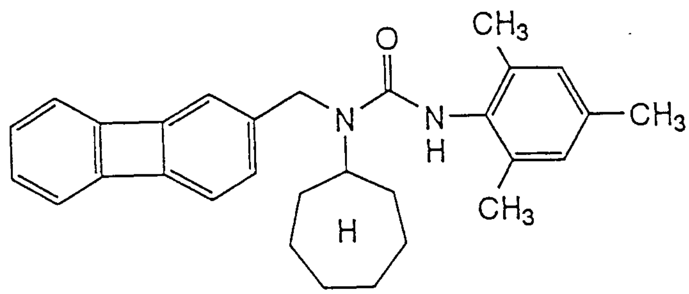

N-Cycloheptyl-(2-biphenylenyl)methylamine(1.10 g, 3.96 mmol) and N-(2,4,6-trimethylphenyl)-0-phenylcarbamate (0.77 g, 3.00 mmol) was refluxed in toluene (10 ml) for 24 hrs. The reaction mixture was then diluted with toluene (50 ml), washed with 1N aqueous sodium hydroxide solution (50 ml x 2 times), dried over anhydrous magnesium sulfate and concentrated under reduced pressure. The residue was purified by silica gel column chromatography to give 0.83 g of the urea compound.

m.p. 123 - 124 °C

IR ($cm^{-1}$, KBr tablet) 1630, 1510, 740

$^1$H-NMR (δ ppm, in deuteriochloroform)

2.05 (6H, s), 2.20 (3H, s), 4.24 (2H, s)

Mass (FAB) m/z 439 ($M^+$ + 1)

The following compounds were synthesized in generally the same manner as above.

EXAMPLE 23

1-Cycloheptyl-1-[1-(2-fluorenyl)ethyl]-3-(2,4,6-trimethylphenyl)urea

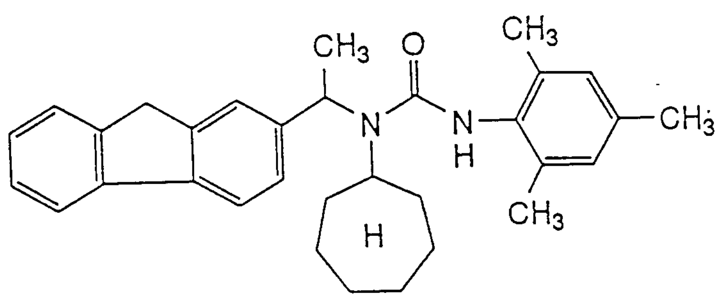

IR ($cm^{-1}$, KBr tablet) 1650, 1500, 1240, 740

$^1$H-NMR (δ ppm, in deuteriochloroform)

2.03 (6H, s), 3.88 (2H, s)

Mass (FAB) m/z 467 ($M^+$ + 1)

EXAMPLE 24

1-(Exo-2-norbornyl)-1-[(9-phenanthrenyl)methyl]-3-[1-(5,6,7,8-tetrahydronaphthyl)]urea

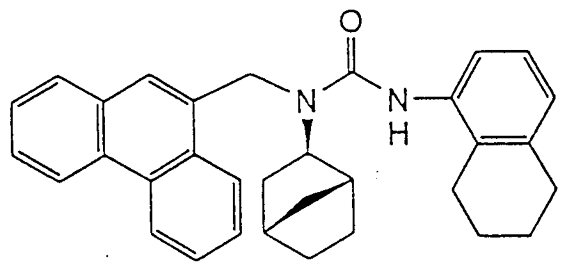

IR ($cm^{-1}$, KBr tablet) 1650, 1530, 1230, 750
$^1$H-NMR ($\delta$ ppm, in deuteriochloroform)
2.60 (2H, m), 5.14 (2H, s)
Mass (FAB) m/z 475 ($M^+$ + 1)

While the invention has been described in detail and with reference to specific embodiments thereof, it will be apparent to one skilled in the art that various changes and modifications can be made therein without departing therefrom.

## Claims

### Claims for the following Contracting States : DE, GB, FR, IT, NL, CH, LI, BE, AT, LU, DK, SE

**1.** A urea derivative of the formula (I) or a salt thereof

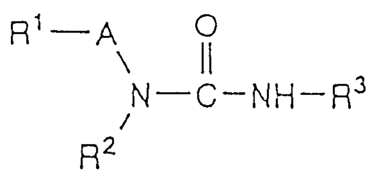

(I)

wherein $R^1$ is a condensed carbocyclic group containing at least 11 carbon atoms; $R^2$ is a cycloalkyl group;

$R^3$ is a tetrahydronaphthyl group or a phenyl group optionally substituted by one or more substituents selected from halogens and $C_1$-$C_5$ alkyl, amino, and mono- and di-($C_1$-$C_5$ alkyl)amino groups; and A is a single bond or an alkylene group containing 1 to 6 carbon atoms.

**2.** A compound according to claim 1 wherein $R^1$ is a fluorenyl group.

**3.** A compound according to claim 1 wherein $R^1$ is a phenanthrenyl group.

**4.** A compound according to any preceding claim wherein A is an alkylene group containing 1 to 6 carbon atoms and $R^2$ is a cycloalkyl group containing 6 to 10 carbon atoms.

**5.** A compound according to any preceding claim wherein $R^3$ is a 2,4,6-trifluorophenyl group.

**6.** A compound according to claim 1, wherein $R^1$ is a fluorenyl group and $R^3$ is a phenyl group substituted by three $C_1$-$C_5$ alkyl groups.

**7.** A compound according to claim 1 which is 1-cycloheptyl-1-(2-flourenylmethyl)-3-(2,4,6-trimethylphenyl)-urea or a salt thereof, or 1-cycloheptyl-1-(9-phenanthrenylmethyl)-3-(2,4,6-trimethylphenyl)ureaor a salt thereof.

**8.** A compound according to claim 1 wherein A is a branched or straight chain alkylene group or $R^2$ is a cycloalkyl group which includes a bridgehead.

**9.** A pharmaceutical composition comprising a therapeutically effective amount of one or more compounds according to any preceding claim with pharmaceutically acceptable carrier.

**10.** A process for producing a urea derivative of formula (I) or a salt thereof

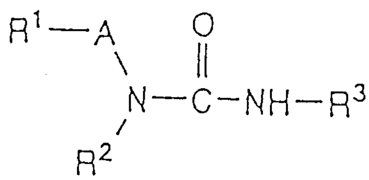

(I)

which comprises reacting compound (II):

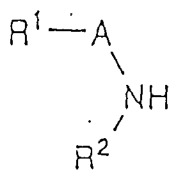

(II)

(a) with haloformic acid ester (III):

$X\text{-}COOR^4$ (III)

and amino compound (IV):

$H_2N\text{-}R^3$ (IV)

concurrently or in any order; or
(b) with isocyanate compound (V)

$R^3\text{-}NCO$ (V);

or
(c) with halogen compound (VI)

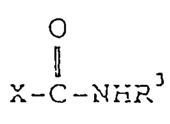

(VI);

wherein $R^1$, $R^2$, $R^3$ and A are as defined in any of claims 1 to 6 and 8, $R^4$ is a phenyl or $C_1$-$C_5$ alkyl group, and X is a halogen atom.

**11.** The use of a compound according to any of claims 1 to 8 for the preparation of a medicament having ACAT inhibitory activity.

**Claims for the following Contracting States : ES, GR**

**1.** A process for producing a urea derivative of formula (I) or a salt thereof

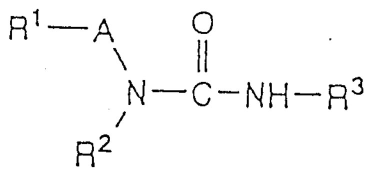

(I)

wherein $R^1$ is a condensed carbocyclic group containing at least 11 carbon atoms; $R^2$ is a cycloalkyl group;

$R^3$ is a tetrahydronapthyl group or a phenyl group optionally substituted by one or more substituents selected from halogens and $C_1$-$C_5$ alkyl, amino, and mono- and di-($C_1$-$C_5$ alkyl)amino groups; and A is a single bond or an alkylene group containing 1 to 6 carbon atoms, which comprises reacting compound (II):

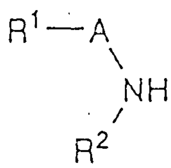

(II)

(a) with haloformic acid ester (III):

X-$COOR^4$ (III)

and amino compound (IV):

$H_2N$-$R^3$ (IV)

concurrently or in any order; or
(b) with isocyanate compound (V)

$R^3$-NCO (V);

or
(c) with halogen compound (VI)

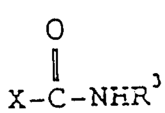

(VI);

wherein
$R^4$ is a phenyl or $C_1$-$C_5$ alkyl group, and X is a halogen atom,

**2.** A process according to claim 1 wherein $R^1$ is a fluorenyl group.

**3.** A process according to claim 1 wherein $R^1$ is a phenanthrenyl group.

**4.** A process according to any preceding claim wherein A is an alkylene group containing 1 to 6 carbon atoms and $R^2$ is a cycloalkyl group containing 6 to 10 carbon atoms.

**5.** A process according to any preceding claim wherein $R^3$ is a 2,4,6-trifluorophenyl group.

6. A process according to claim 1, wherein $R^1$ is a fluorenyl group and $R^3$ is a phenyl group substituted by three $C_1$-$C_5$ alkyl groups.

7. A process according to claim 1 wherein the product is 1-cycloheptyl-1-(2-fluorenylmethyl)-3-(2,4,6-trimethylphenyl)urea or a salt thereof, or 1-cycloheptyl-1-(9-phenanthrenylmethyl)-3-(2,4,6-trimethylphenyl)ureaor a salt thereof.

8. A process according to claim 1 wherein A is a branched or straight chain alkylene group or $R^2$ is a cycloalkyl group which includes a bridgehead.

9. A method of making a pharmaceutical composition which comprises combining with pharmaceutically acceptable carrier a therapeutically effective amount of at least one compound selected from compounds of formula (I) as defined in any preceding claim and salts thereof.

10. The use of a product compound of formula I as defined in any of claims 1 to 8 or salt thereof for the preparation of a medicament having ACAT inhibitory activity.

**Patentansprüche**

**Patentansprüche für folgende Vertragsstaaten : DE, GB, FR, IT, NL, CH, LI, BE, AT, LU, DK, SE**

1. Harnstoffderivat der Formel (I) oder ein Salz davon

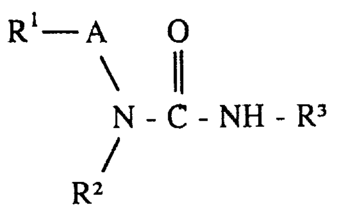

(I)

worin $R^1$ eine kondensierte carboxyclische Gruppe mit mindestens 11 Kohlenstoffatomen ist; $R^2$ eine Cycloalkylgruppe ist; $R^3$ für eine Tetrahydronaphthylgruppe oder eine Phenylgruppe steht, gegebenenfalls durch ein oder mehrere aus Halogenen und $C_1$-$C_5$-Alkyl-, Amino- und Mono- und Di-($C_1$-$C_5$-alkyl)-aminogruppen ausgewählte Substituenten substituiert; und worin A eine Einfachbindung oder eine Alkylengruppe mit 1 bis 6 Kohlenstoffatomen ist.

2. Verbindung gemäß Anspruch 1, worin $R^1$ eine Fluorenylgruppe ist.

3. Verbindung gemäß Anspruch 1, worin $R^1$ eine Phenanthrenylgruppe ist.

4. Verbindung gemäß mindestens einem der vorhergehenden Ansprüche, worin A eine Alkylengruppe mit 1 bis 6 Kohlenstoffatomen und $R^2$ eine Cycloalkylgruppe mit 6 bis 10 Kohlenstoffatomen ist.

5. Verbindung gemäß mindestens einem der vorhergehenden Ansprüche, worin $R^3$ eine 2,4,6-Trifluorphenylgruppe ist.

6. Verbindung gemäß Anspruch 1, worin $R^1$ eine Fluorenylgruppe und $R^3$ eine Phenylgruppe, substituiert durch drei $C_1$-$C_5$-Alkylgruppen, ist.

7. Verbindung gemäß Anspruch 1, welche 1-Cycloheptyl-1-(2-fluorenylmethyl)-3-(2,4,6-trimethylphenyl)-harnstoff oder ein Salz davon oder 1-Cycloheptyl-1-(9-phenanthrenylmethyl)-3-(2,4,6-trimethylphenyl)-harnstoff oder ein Salz davon ist.

8. Verbindung gemäß Anspruch 1, worin A eine verzweigte oder geradkettige Alkylengruppe oder $R^2$ eine Cycloalkylgruppe ist, welche einen Brückenkopf einschließt.

9. Pharmazeutische Zusammensetzung, umfassend eine therapeutisch wirksame Menge einer oder mehrerer Verbindungen gemäß mindestens einem der vorhergehenden Ansprüche mit einem pharmazeutisch annehmbaren Träger.

10. Verfahren zur Herstellung eines Harnstoffderivats der Formel (I) oder eines Salzes davon

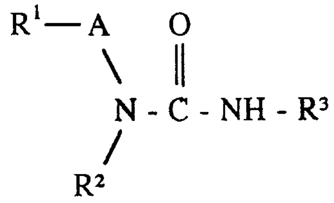

(I)

welches das Umsetzen der Verbindung (II)

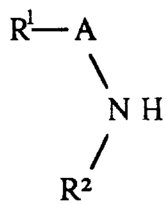

(II)

(a) mit Halogenameisensäureester (III):

X - $COOR^4$ (III)

und einer Aminoverbindung (IV):

$H_2N$ - $R^3$ (IV)

gleichzeitig oder in beliebiger Reihenfolge; oder
(b) mit einer Isocyanatverbindung (V)

$R^3$ - NCO (V);

oder
(c) mit einer Halogenverbindung (VI)

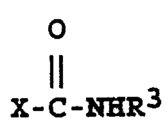

(VI)

umfaßt, worin $R^1$, $R^2$, $R^3$ und A wie in mindestens einem der Ansprüche 1 bis 6 und 8 definiert ist, $R^4$ eine Phenyl- oder $C_1$-$C_5$-Alkylgruppe ist und X für ein Halogenatom steht.

11. Verwendung einer Verbindung gemäß mindestens einem der Ansprüche 1 - 8 zur Herstellung eines Medikamentes mit ACAT-Inhibitionsaktivität.

**Patentansprüche für folgende Vertragsstaaten : ES, GR**

1. Verfahren zur Herstellung eines Harnstoffderivats der Formel (I) oder eines Salzes davon

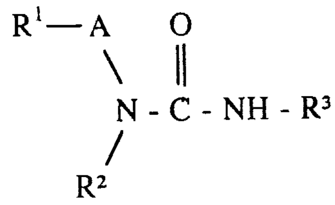

(I)

worin $R^1$ eine kondensierte carboxyclische Gruppe mit mindestens 11 Kohlenstoffatomen ist; $R^2$ eine Cycloalkylgruppe ist; $R^3$ für eine Tetrahydronaphthylgruppe oder eine Phenylgruppe steht, gegebenenfalls durch ein oder mehrere aus Halogenen und $C_1$-$C_5$-Alkyl-, Amino- und Mono- und Di-($C_1$-$C_5$-alkyl)-aminogruppen ausgewählte Substituenten substituiert; und worin A eine Einfachbindung oder eine Alkylengruppe mit 1 bis 6 Kohlenstoffatomen ist, welches das Umsetzen der Verbindung (II)

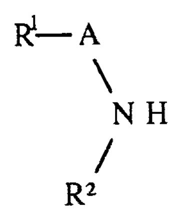

(II)

(a) mit Halogenameisensäureester (III):

X - $COOR^4$ (III)

und einer Aminoverbindung (IV):

$H_2N$ - $R^3$ (IV)

gleichzeitig oder in beliebiger Reihenfolge; oder
(b) mit einer Isocyanatverbindung (V)

$R^3$ - NCO (V);

oder
(c) mit einer Halogenverbindung (VI)

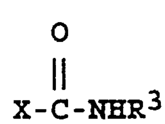

(VI)

umfaßt, worin $R^4$ eine Phenyl- oder $C_1$-$C_5$-Alkylgruppe ist und X für ein Halogenatom steht.

2. Verfahren gemäß Anspruch 1, worin $R^1$ eine Fluorenylgruppe ist.

3. Verfahren gemäß Anspruch 1, worin $R^1$ eine Phenanthrenylgruppe ist.

4. Verfahren gemäß mindestens einem der vorhergehenden Ansprüche, worin A eine Alkylengruppe mit 1 bis 6 Kohlenstoffatomen und $R^2$ eine Cycloalkylgruppe mit 6 bis 10 Kohlenstoffatomen ist.

**5.** Verfahren gemäß mindestens einem der vorhergehenden Ansprüche, worin $R^3$ eine 2,4,6-Trifluorphenylgruppe ist.

**6.** Verfahren gemäß Anspruch 1, worin $R^1$ eine Fluorenylgruppe und $R^3$ eine Phenylgruppe, substituiert durch drei $C_1$-$C_5$-Alkylgruppen, ist.

**7.** Verfahren gemäß Anspruch 1, worin das Produkt 1-Cycloheptyl-1-(2-fluorenylmethyl)-3-(2,4,6-(trimethylphenyl)harnstoff oder ein Salz davon oder 1-Cycloheptyl-1-(9-phenanthrenylmethyl)-3-(2,4,6-trimethylphenyl)harnstoff oder ein Salz davon ist.

**8.** Verfahren gemäß Anspruch 1, worin A eine verzweigte oder geradkettige Alkylengruppe oder $R^2$ eine Cycloalkylgruppe ist, welche einen Brückenkopf einschließt.

**9.** Verfahren zur Herstellung einer pharmazeutische Zusammensetzung, umfassend das Kombinieren einer therapeutisch wirksamen Menge einer oder mehrerer Verbindungen (I) gemäß mindestens einem der vorhergehenden Ansprüche und Salze derselben mit einem pharmazeutisch annehmbaren Träger.

**10.** Verwendung einer Produktverbindung der Formel I gemäß mindestens einem der Ansprüche 1 - 8 oder eines Salzes derselben zur Herstellung eines Medikamentes mit ACAT-Inhibitionsaktivität.

**Revendications**

**Revendications pour les Etats contractants suivants : DE, GB, FR, IT, NL, CH, LI, BE, AT, LU, DK, SE**

**1.** Un dérivé d'urée de formule (I) ou un sel de celui-ci

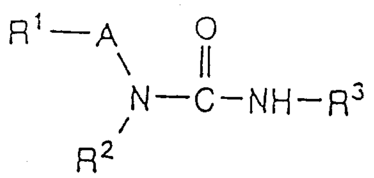

(I)

dans laquelle $R^1$ est un groupe carbocyclique condensé contenant au moins 11 atomes de carbone ; $R^2$ est un groupe cycloalkyle ; $R^3$ est un groupe tétrahydronaphtyle ou un groupe phényle optionnellement substitué par un ou plusieurs substituants choisis parmi des halogènes et des groupes alkyle en $C_1$-$C_5$, amino et mono- et di-(alkyl en $C_1$-$C_5$)amino ; et A est une liaison simple ou un groupe alkylène contenant 1 à 6 atomes de carbone.

**2.** Un composé selon la revendication 1 dans lequel $R^1$ est un groupe fluorenyle.

**3.** Un composé selon la revendication 1, dans lequel $R^1$ est un groupe phénanthrenyle.

**4.** Un composé selon une quelconque revendication précédente, dans lequel A est un groupe alkylène contenant 1 à 6 atomes de carbone et $R^2$ est un groupe cycloalkyle contenant 6 à 10 atomes de carbone.

**5.** Un composé selon une quelconque revendication précédente, dans lequel $R^3$ est un groupe 2,4,6-trifluorophényle.

**6.** Un composé selon la revendication 1, dans lequel $R^1$ est un groupe fluorényle et $R^3$ est un groupe phényle substitué par trois groupes alkyle en $C_1$-$C_5$.

**7.** Un composé selon la revendication 1, qui est la 1-cycloheptyl-1-(2-fluorénylméthyl)-3-(2,4,6-triméthylphényl)urée ou un sel de celle-ci, ou la 1-cycloheptyl-1-(9-phénanthrénylméthyl)-3-(2,4,6-triméthylphényl)urée ou un sel de celle-ci.

**8.** Un composé selon la revendication 1, dans lequel A est un groupe alkylène à chaîne ramifiée ou linéaire ou $R^2$ est un groupe cycloalkyle qui inclut une tête de pont.

**9.** Une composition pharmaceutique comprenant une quantité thérapeutiquement efficace d'un ou plusieurs composés selon une quelconque revendication précédente avec un support pharmaceutiquement acceptable.

**10.** Un procédé de fabrication d'un dérivé d'urée de formule (I) ou d'un sel de celui-ci

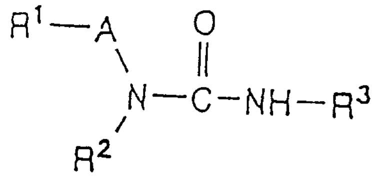

(I)

qui comprend la réaction d'un composé (II)

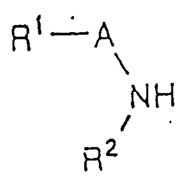

(II)

(a) avec un ester d'acide haloformique (III) :

$X\text{-}COOR^4$ (III)

et un composé amino (IV) :

$H_2N\text{-}R^3$ (IV)

concurremment ou dans n'importe quel ordre ; ou
(b) avec un composé isocyanate (V)

$R^3\text{-}NCO$ (V);

ou
(c) avec un composé halogéné (VI)

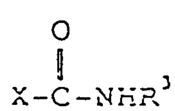

(VI) ;

où $R^1$, $R^2$, $R^3$ et A sont tels que définis dans l'une des revendications 1 à 6 et 8, $R^4$ est un groupe phényle ou alkyle en $C_1$-$C_5$ et X est un atome d'halogène.

**11.** L'utilisation d'un composé selon l'une quelconque des revendications 1 à 8 pour la préparation d'un médicament ayant une activité inhibitrice de l'ACAT.

**Revendications pour les Etats contractants suivants : ES, GR**

**1.** Un procédé de fabrication d'un dérivé d'urée de formule (I) ou un sel de celui-ci

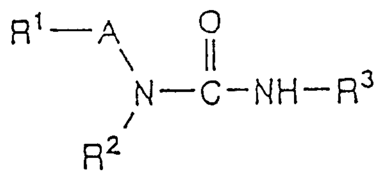

(I)

dans lequel $R^1$ est un groupe carbocyclique condensé contenant au moins 11 atomes de carbone ; $R^2$ est un groupe cycloalkyle ; $R^3$ est un groupe tétrahydronaphtyle ou un groupe phényle optionnellement substitué par un ou plusieurs substituants choisis parmi des halogènes et des groupes alkyle en $C_1$-$C_5$, amino et mono- et di-(alkyl en $C_1$-$C_5$)amino ; et A est une liaison simple ou un groupe alkylène contenant 1 à 6 atomes de carbone, qui comprend la réaction d'un composé (II) :

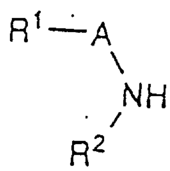

(II)

(a) avec un ester d'acide haloformique (III) :

$X\text{-}COOR^4$ (III)

et un composé amino (IV) :

$H_2N\text{-}R^3$ (IV)

concurremment ou dans un ordre quelconque ; ou
(b) avec un composé isocyanate (V)

$R^3\text{-}NCO$ (V) ;

ou
(c) avec un composé halogéné (VI)

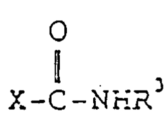

(VI) ;

où
$R^4$ est un groupe phényle ou alkyle en $C_1$-$C_5$ et X est un atome d'halogène.

**2.** Un procédé selon la revendication 1 dans lequel $R^1$ est un groupe fluorényle.

**3.** Un procédé selon la revendication 1, dans lequel $R^1$ est un groupe phénanthrényle.

**4.** Un procédé selon une quelconque revendication précédente, dans lequel A est un groupe alkylène contenant 1 à 6 atomes de carbone et $R^2$ est un groupe cycloalkyle contenant 6 à 10 atomes de carbone.

**5.** Un procédé selon une quelconque revendication précédente, dans lequel $R^3$ est un groupe 2,4,6-trifluorophényle.

**6.** Un procédé selon la revendication 1, dans lequel $R^1$ est un groupe fluorényle et $R^3$ est un groupe phényle substitué par trois groupes alkyle en $C_1$-$C_5$.

**7.** Un procédé selon la revendication 1, dans lequel le produit est la 1-cycloheptyl-1-(2-fluorénylméthyl)-3-(2,4,6-triméthylphényl)urée ou un sel de celle-ci, ou la 1-cyclohepty-1-(9-phénanthrénylméthyl)-3-(2,4,6-triméthylphényl)urée ou un sel de celle-ci.

**8.** Un procédé selon la revendication 1, dans lequel A est un groupe alkylène à chaîne ramifiée ou linéaire ou $R^2$ est un groupe cycloalkyle qui inclut une tête de pont.

**9.** Un procédé de fabrication d'une composition pharmaceutique qui comprend une combinaison d'un support pharmaceutiquement acceptable avec une quantité thérapeutiquement efficace d'au moins un composé choisi parmi les composés de formule (I) tels que définis dans une quelconque revendication précédente et les sels de ceux-ci.

**10.** L'utilisation d'un composé de produit de formule (I) tel que défini dans l'une quelconque des revendications 1 à 8 ou un sel de celui-ci pour la préparation d'un médicament ayant une activité inhibitrice de l'ACAT.